# EUROPEAN PATENT APPLICATION

(11) **EP 3 932 472 A1**
(43) Date of publication of application: **05.01.2022**
(21) Application number: 19879435.6
(22) Date of filing: 25.10.2019
(51) Int. Cl.: A61N 1/32

(54) **DEVICE FOR TREATING NEURODEGENERATIVE DISEASES**

(30) Priority: 30.10.2018 ES 201831649 U
(71) Applicant: Virbeco Solutions, S.L., 08226 Terrasa (Barcelona) (ES)
(72) Inventor: MORAL VICO, Miguel Angel, 08226 Terrassa (Barcelona) (ES)
(74) Representative: Durán-Corretjer, S.L.P.
(86) International application number: PCT/ES2019/070725
(87) International publication number: WO 2020/089496

(57) **Abstract**

Device for the treatment of degenerative diseases involving inflammation, which includes an electric current generator comprising an electric current modulator for the generator, at least one active electrode and at least one return electrode through which currents are applied, with these electrodes being connected to the generator by wires in which the currents applied have a damped current form, and with these currents applied having a frequency of between 60 and 70 kHz and these currents applied being modulated via a sinusoidal signal of between 80 and 120 Hz.

## Description

This application relates to a device for the treatment of degenerative diseases.

Degenerative diseases are a type of disease involving a continuous degeneration of cells. Cellular degeneration is a serious problem for those individuals affected, causing the functioning and/or structure of tissues and organs affected by this cellular degeneration to deteriorate over time. These diseases are associated with each individual's pace of life and affect a high percentage of the population. Furthermore, they are usually chronic, and cellular degeneration usually affects the individual over the course of their lifetime.

Many of these degenerative diseases involve inflammation, e.g. macular degeneration, prostatitis or fistulas. When a disease involves inflammation, a number of cytokines are affected which are considered to be a marker of this inflammation.

Macular degeneration, or age-related macular degeneration, is a disease that causes lesions in the central section of the retina known as the macula. The macula is the section of the eye that allows individuals to see details clearly, as it is responsible for central vision, which is required for reading, driving or watching television. It is not painful, but it causes degeneration and even the death of the macular cells.

Macular degeneration can be wet or dry. The wet form occurs when abnormal blood vessels grow under the macula, often leaking blood and fluid and forming a neovascular membrane. In this case, the damage to the macula occurs rapidly. The dry form, however, occurs when the light-sensitive cells of the macula deteriorate and the central vision is lost progressively over the years.

Many of the treatments currently performed in ophthalmology centres are based on laser treatments, or on intravitreal injections with anti-angiogenic factors to treat wet macular degeneration. These intravitreal injections block the progression of the neovascular membranes. There is no treatment that is really effective for dry macular degeneration beyond the surgical implantation of telescopic lenses in the patient's eye, and in many cases the patient is advised to maintain a healthier lifestyle.

The application of currents using electrodes is a known method for muscular stimulation, but there is no state-of-the-art effective treatment for degenerative diseases.

The Australian Patent application AU 2016323117 A1 involves the disclosure of an electrotherapy device comprising various electrodes configured for stimulation of a patient via microcurrents designed for ocular stimulation. The US Patent application US 2005137649 A1 involves the disclosure of an electrotherapy device for the treatment of macular degeneration using a conductive patch that is placed over the eye socket without being in direct contact with the eyelids. At least two microcurrent signals previously modulated with different modulation are applied to these electrodes, with these microcurrents being undamped. The frequency modulation applied in this patent, decreasing in time starting at high frequencies and with a range of between 400 Hz and 0.1 Hz, has not provided effective results. In addition to this, these inventions are not effective in the treatment of non-ocular degenerative diseases.

The patent for the present invention involves the disclosure of an electrotherapy device for the treatment of degenerative diseases with a modulation that works and which enables more effective treatment than the previous state-of-the-art documents, enabling a reduction in degenerative diseases such as macular degeneration, prostatitis and fistulas. This treatment is effective in the modulation range used and disclosed in this invention.

The partial reversal of symptoms has been confirmed via experiments to be due to a rapid reduction in adjacent inflammation.

More specifically, this invention reveals a new modulation of an electrotherapy device for the treatment of degenerative diseases which is more effective than those in the previous state-of-the-art.

More specifically, this invention involves the disclosure of an electrotherapy device for the treatment of degenerative diseases involving inflammation, which includes an electrotherapy device for the treatment of degenerative diseases involving inflammation, including an electric current generator which comprises an electric current modulator for the generator, at least one active electrode and at least one return electrode through which currents are applied, with these electrodes being connected to the generator by wires, characterised by the fact that the currents applied have a damped current form, and with these currents applied having a frequency of between 60 and 70 kHz and these currents applied being modulated via a sinusoidal signal of between 80 and 120 Hz. Preferably, the current applied is modulated via a sinusoidal signal at 100 Hz. Even more preferably, the current applied has a frequency of 66 kHz. More preferably, the device is intended for the treatment of macular degeneration.

Preferentially, the damped current is between 35 and 45 kVpp. Preferentially, this current is 40 kVpp. In a preferential configuration, the current applied is applied with a repetition of between 4.5 ms and 6.5 ms. More preferentially, with a repetition of between 5.6 ms.

Preferentially, the return electrode is in the form of a blanket or is located inside a textile element. Preferably, the active electrodes are made of conductive rubber. In an even more preferable embodiment, the active electrodes have a curvature on a lower part of these adapted for positioning of these active electrodes on the eyelids. Preferably, the electrotherapy device comprises two active electrodes exactly. Even more preferably, the wires which connect the active electrodes to the generator are located on these active electrodes in holes in the electrodes intended for this arrangement.

In a preferential embodiment, the active electrodes are arranged in a mask with holes prepared for placement of the electrodes in these. Preferably, the mask is made of non-conductive material. More preferably, the mask is made of silicone. More preferably, the mask has guides on each side of the mask to facilitate the placement of the wiring in the mask. Even more preferably, the different wires in the device are colour-coded in accordance with their function for improved identification of these wires. Even more preferably, the mask has a mechanism for adjusting to the contour of a head via adjustable connection mechanisms. Preferably, this adjustable connection mechanism is an adhesive seam. Even more preferably, this adjustable connection mechanism is commercial Velcro.

In one embodiment of the invention, the electrotherapy device is controlled by equipment comprising a display, an RFID card reader and a Bluetooth module. Preferably, the display of the equipment in the invention is a touch display.

For improved comprehension, some drawings of an embodiment of this invention are attached for explanatory but not limiting purposes.
Figure 1 shows an example of connection of a device in accordance with the present invention.
Figure 2 shows an example of modulation of a waveform in accordance with the present invention.
Figure 3 shows a perspective view of an example embodiment of a mask for the placement of active electrodes in accordance with the present invention.
Figure 4 shows a top view of the mask from figure 3.
Figure 5 shows a raised view of the mask from figures 3 and 4 with the mask open.
Figure 6 shows a top view of the mask from figures 3, 4 and 5 with the mask open and without electrodes.
Figure 7 shows a perspective view of electrodes in accordance with the present invention.
Figure 8 shows a raised side view of the electrodes from figure 7.

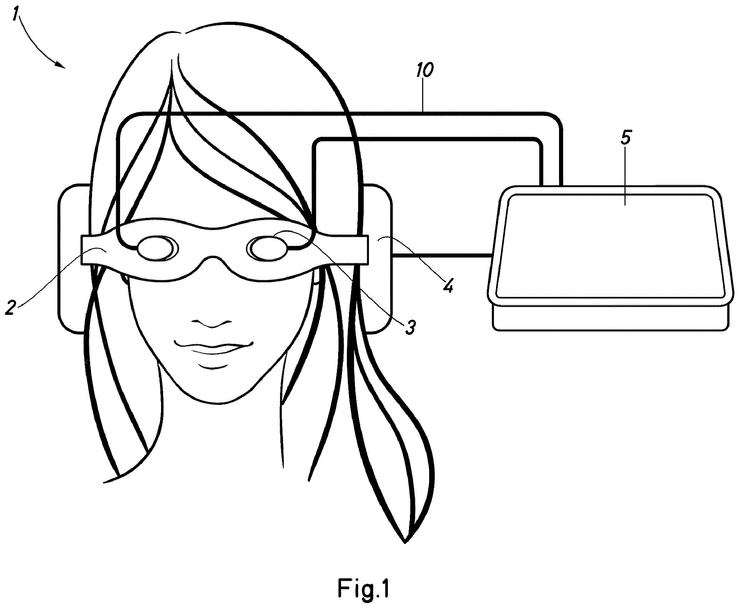

Figure 1 shows an example embodiment of an electrotherapy device -1- for the treatment of degenerative diseases involving inflammation, in particular macular degeneration, via which currents are applied to an area of the skin that needs to be treated. This device comprises an electric current generator -5- which comprises a modulator for the electric current generator -5-, at least one active electrode -3- and at least one return electrode -4- via which currents are applied. The device shown in the figure is intended to treat macular degeneration, and has two active electrodes -3- that are placed on the user's eyelids using a mask -2- as a support. The active electrodes -3- are located one above each of the eyelids.

The active electrodes -3- adjust to the shape of the human eye thanks to a curvature on the lower part adjusted for placement on the eyelids. This shape provides effective contact between the active electrode -3- and the eyelid, allowing transmission of the modulated wave to the affected area.

The connection for the device -1- is made by connecting the active electrodes -3- to an electric current generator -5- comprising a modulator of the generator's electric current via wires -10-. In addition to this, the device may have a return electrode -4- connected to the electric current generator -5- and which acts as a resistive load. Once the active electrodes -3- and this return electrode -4- are in contact with the user's body, the electric circuit is closed.

In figure 1, the return electrode -4- is in the form of a blanket and is located on the head of a chair under the user's head, making contact with the user's head. A return electrode can also be used that is located in a blanket or other textile element. This arrangement allows the active electrodes -3- and the return electrode -4- to be connected to the user's body, creating a straight line connection between the three elements. The straight line connection facilitates the flow of current while ensuring in turn that the electric current comes into contact with the eyelids through the active electrode -3- and leaves the body through the return electrode -4-. The blanket form for the return electrode -4- is an optimal and comfortable shape in establishing contact between the return electrode -4- and the body.

One of the main features in this device is the modulation of the applied currents. According to the present invention, the application of currents to the area affected by a degenerative disease not only reduces the progression of this disease, but also allows part of the lost vision to be recovered.

The effectiveness of any treatment for macular degeneration depends primarily on the waveform of the current applied. The application of currents over the affected area can only treat these diseases effectively with proper modulation.

The treatment is only effective at particular frequencies. Obtaining proper modulation of the waveform for the applied current is therefore absolutely necessary for the treatment.

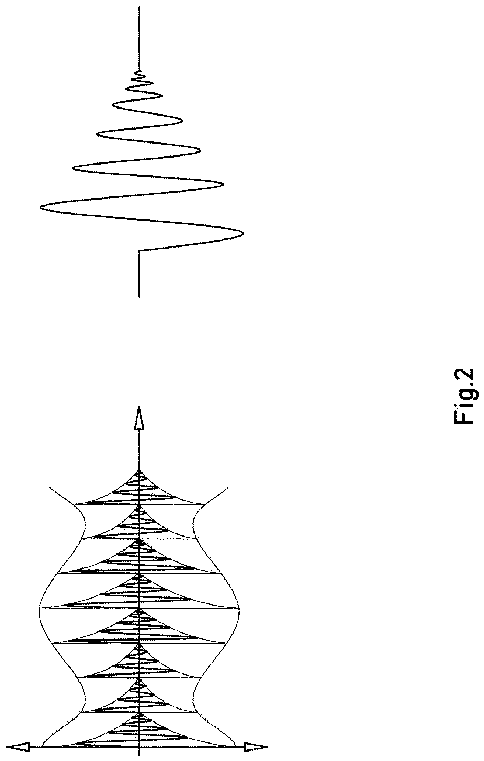

The current applied to the active electrodes -3- for the treatment of macular degeneration is a current in the form of a damped current and which is generated by the electric current generator -5- and modulated by a modulator for the generator's electric current -5- which is contained in this generator -5-. In the example embodiment in the figures, the current applied to the active electrodes -3- and which is shown in figure 2, which is the modulator output current, has a frequency of 66 kHz and is applied with a repetition of 5.6 ms. This signal is modulated via a sinusoidal signal at 100 Hz. This damped current is 40 kVpp (from peak to peak).

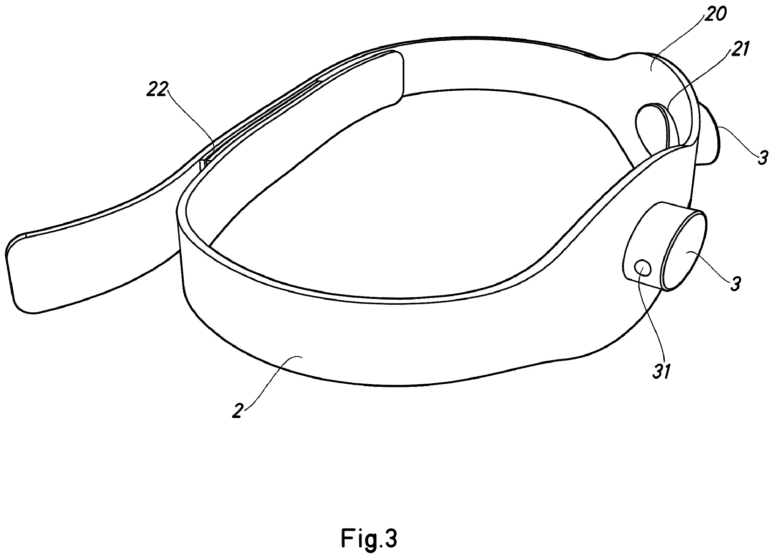

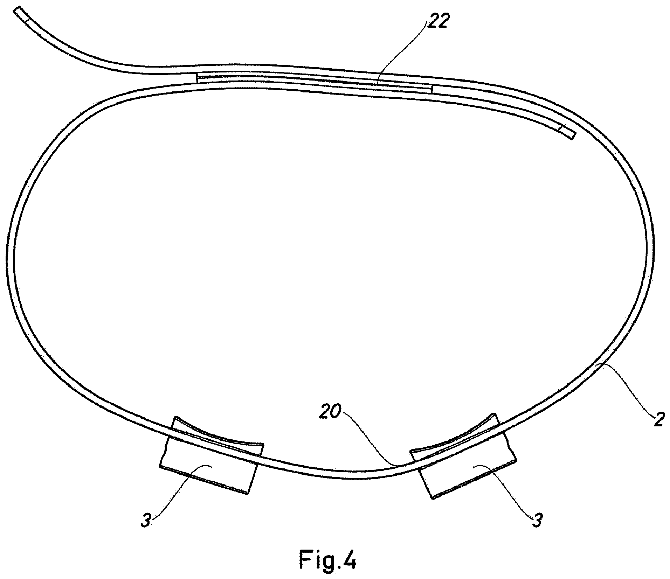

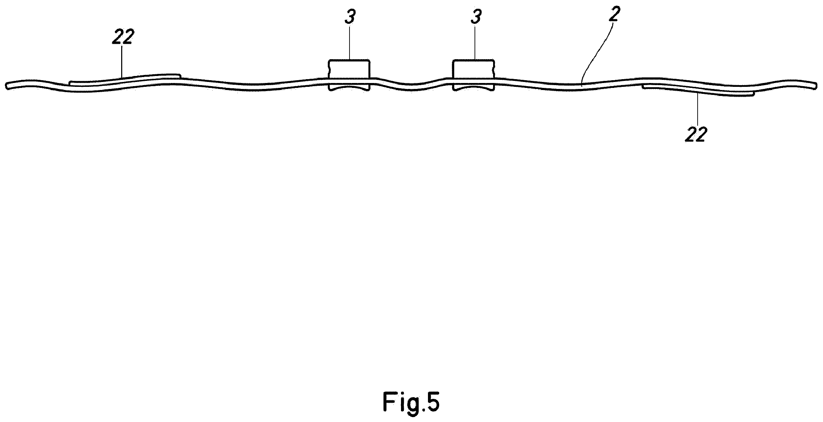

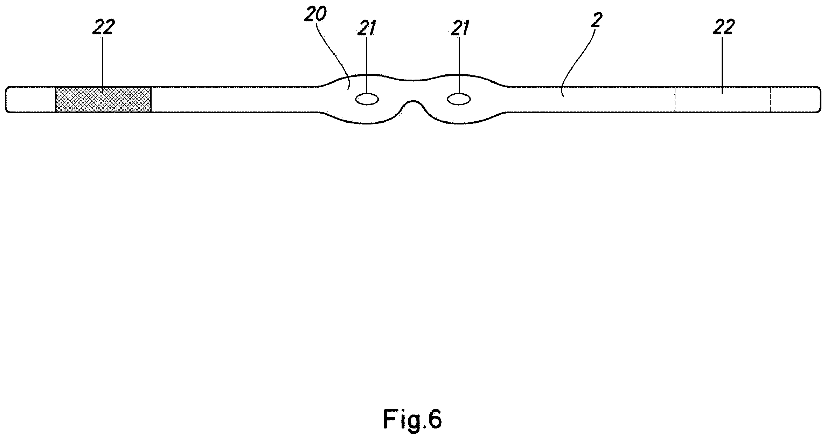

In the example in the figures, the active electrodes -3- are positioned on the eyelids using a mask -2-. The placement of these electrodes on the mask is shown in figures 3, 4, 5 and 6. Figures 3 and 4 show the active electrodes -3- located on the mask -2- in perspective and top views respectively. Figure 5 shows a raised view of the mask and electrode assembly and figure 6 shows a top view of the mask without the electrodes.

The eye mask -2- is an element with non-conductive material, preferably silicone, having the shape of an extended elastic strip adjusted to the morphology of the contour of a human head, and with two rounded areas -20- in one area of this prepared for contact with the user's eye area. In the mask in figure 3, these areas -20- are located in the central part of the mask -2- and the mask is of equal length on each side of the mask, although this length can be variable and the mask can be asymmetrical. The mask -2- has holes -21- in these rounded areas -20- for housing the active electrodes -3-, with these holes -21- being in contact with the electrodes -3- via guides -32- located on the contour of the electrodes and adjusted for this purpose. In addition to this, the mask -2- can have guides on each side of the mask to facilitate the placement of the wiring -10- in the mask.

The mask -2- shown in the figures has a mechanism for adjusting to the contour of a head via an adjustable connection mechanism -22-. This adjustable connection mechanism allows the same mask to be used by different users, irrespective of the circumference of their heads. In the embodiment shown in the figures, this mechanism is a Velcro-type adhesive tape. Alternatively, any other type of connection mechanism can be used.

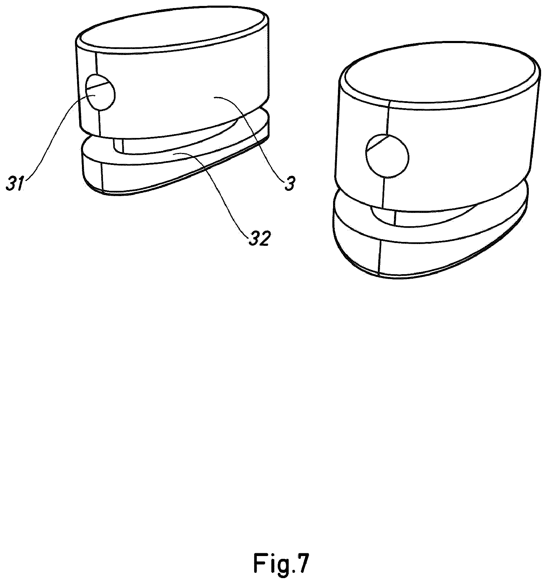

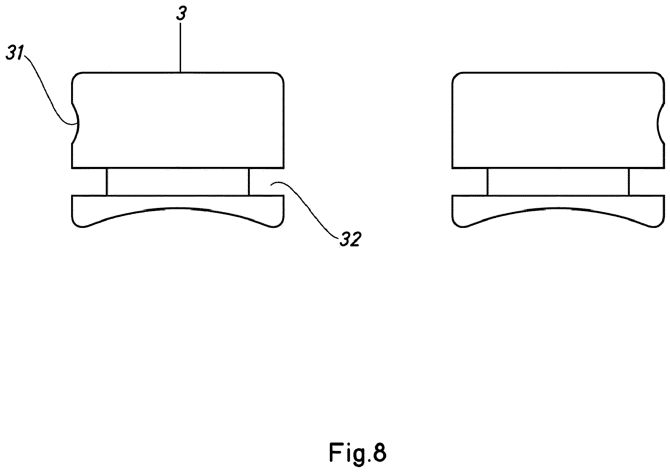

The active electrodes -3- used in the embodiment in the figures are shown in detail in figures 7 and 8. The active electrodes are made of conductive rubber and have holes -31- in which the tip of the wires-10- which connect the active electrode -3- to the electric current generator -5- are placed. In addition to this, the active electrodes -3- have guides -32- on the contour of the electrodes for contact of these and their subsequent placement in the holes -21- of the mask -2-.

A preferential process for treating macular degeneration using the device disclosed in this example is as follows:
- place the return electrode -4-, in the form of a return blanket, on a backrest, chair or similar item and connect the return electrode -4- to the electric current generator -5-;
- place a towel or dry protection over the return electrode -4- to prevent the current from flowing to unwanted areas;
- place the active electrodes -3- inside the mask -2- into the holes -21- of the mask designated for this purpose;
- place the wiring -10- in the holes -31- of the active electrodes -3-, with the tip of each wire being in contact with these electrodes;
- apply a conductive gel to each active electrode -3-;
- place the user on top of the return electrode -4- and place the mask -2- on this user with the eyelids closed or in such a way that the active electrodes -3- are placed on top of the eyelids. The wiring -10-must be located away from the face;
- fasten both sides of the mask -2- down to prevent the mask -2- from moving during treatment. Preferably, adjusting the circumference of the mask -2- using the connection methods -22- incorporated in the mask;
- connect the active electrodes -3- to the equipment of the electric current generator -5-.

The device in the invention enables a completely harmless treatment to be offered to the user, and is prepared for application in an indeterminate number of sessions.

The modulation of the current applied is suitable for treating macular degeneration. A study was carried out of the biochemical alteration of some proteins related to human ophthalmological inflammatory processes following application of the wave with the modulation in this example.

This experiment involved using a commercial kit to analyse the expression of 34 cytokines involved in inflammation. These cytokines are related to the inflammation process and are used as a marker for this inflammation. A technique known as Human Inflammation Antibody Array was used, which is based on the use of small nitrocellulose membranes which contain a large variety of antibodies impregnated on their surface, more specifically 34 different antibodies. A marker such as a fluorescent one was associated with each antibody. The higher the intensity of the signal received from each cytokine by each marker, the higher the inflammation analysed.

A total of 6 samples were assessed for this purpose: 5 samples of human tears obtained from healthy people and ophthalmology patients, plus a control sample with lysis buffer, with the results obtained before and after the treatment then compared.

The process followed to prepare the samples and obtain the results is detailed below:
- homogenise the samples with a buffer that contains protease inhibitors;
- measure the total protein concentration of the homogenised samples;
- complete the relevant dilutions to achieve the same amount of total protein for each pair of samples to be tested, both before and after the treatment;
- use the Human Inflammation Antibody Array technique for simultaneous detection of inflammation-related factors (proteins);
- complete the relevant incubations and show the arrays;
- analyse the results with a computer programme.

Each membrane used had a total of 96 positions arranged in the form of a grid, with 68 positions (corresponding to the 34 antibodies in duplicate) corresponding with positions that contained a fixed antibody and that were designed to detect a single protein, and with the remaining 28 being positive, negative and blank controls.

The intensity values for the cytokines obtained and compared before and after treatment made it possible to ensure that the waveform used in this example embodiment (applied with a repetition of 5.6 ms, modulated via a sinusoidal signal at 100 Hz and also modulated in such a way that the modulator output current has a frequency of 66 kHz, with this damped current being 40 kVpp) acts directly on the cytokines acting as markers of the inflammation, reducing the intensity of the signal received, and therefore reducing the inflammation.

Although the invention has been described and depicted based on various representative examples, it should be understood that these example embodiments are in no way limiting for the present invention, with the result that any of the variations that remain included directly or by way of equivalence in the content of the attached claims shall be considered to be included within the scope of the present invention.

## Claims

1. Device for the treatment of degenerative diseases involving inflammation, which includes an electric current generator comprising an electric current modulator for the generator, at least one active electrode and at least one return electrode through which currents are applied, with these electrodes being connected to the generator by wires, **characterised by** the fact that the currents applied have a damped current form, and with these currents applied having a frequency of between 60 and 70 kHz and these currents applied being modulated via a sinusoidal signal of between 80 and 120 Hz.

2. Device in accordance with claim 1, **characterised by** the fact that the current applied is modulated via a sinusoidal signal at 100 Hz.

3. Device in accordance with any of the preceding claims, **characterised by** the fact that the current applied has a frequency of 66 kHz.

4. Device in accordance with any of the preceding claims, **characterised by** the fact that the damped current is between 35 and 45 kVpp.

5. Device in accordance with any of the preceding claims, **characterised by** the fact that the damped current is 40 kVpp.

6. Device in accordance with any of claims 2 to 6, **characterised by** the fact that the current applied is applied with a repetition of between 4.5 ms and 6.5 ms.

7. Device in accordance with any of claims 2 to 7, **characterised by** the fact that the current applied is applied with a repetition of between 5.6 ms.

8. Device in accordance with any of the preceding claims, **characterised by** the fact that the return electrode is in the form of a blanket or is located inside a textile element.

9. Device in accordance with any of the preceding claims, **characterised by** the fact that the active electrodes are made of conductive rubber.

10. Device in accordance with any of the preceding claims, **characterised by** the fact that the active electrodes have a curvature on a lower part of these adapted for positioning of these active electrodes on the eyelids.

11. Device in accordance with any of the preceding claims, **characterised by** the fact that it comprises two active electrodes exactly.

12. Device in accordance with any of the preceding claims, **characterised by** the fact that the wires which connect the active electrodes to the electric current generator are located on these active electrodes in holes in the electrodes intended for this arrangement.

13. Device in accordance with any of the preceding claims, **characterised by** the fact that the active electrodes are arranged in a mask with holes prepared for placement of the electrodes in these.

14. Device in accordance with claim 13, **characterised by** the fact that the mask is made of non-conductive material.

15. Device in accordance with claim 14, **characterised by** the fact that the mask is made of silicone.

16. Device in accordance with any of claims 13 to 15, **characterised by** the fact that the mask has a mechanism for adjusting to the contour of a head via adjustable connection mechanisms.

17. Device in accordance with the preceding claim, **characterised by** the fact that the adjustable connection mechanism is an adhesive seam.

18. Device in accordance with any of the preceding claims, **characterised by** the fact that the device is controlled by equipment comprising a display, an RFID card reader and a Bluetooth module.
